# EUROPÄISCHE PATENTSCHRIFT

(11) **EP 1 719 483 B1**
(45) Veröffentlichungstag und Bekanntmachung des Hinweises auf die Patenterteilung: **10.08.2011**
(21) Anmeldenummer: 05009616.3
(22) Anmeldetag: 02.05.2005
(51) Int. Cl.: A61F 9/01

(54) **Verfahren zur Steuerung eines Lasers für die Ablation einer Hornhautschicht eines Auges**
Method of controlling a laser for ablating a corneal layer
Méthode de contrôle d'un laser permettant l'ablation d'une couche de la cornée

(43) Veröffentlichungstag der Anmeldung: 08.11.2006
(73) Patentinhaber: Schwind eye-tech-solutions GmbH & Co. KG, 63801 Kleinostheim (DE)
(72) Erfinder: Carriazo, Cesar C., Dr., Baranquilla (CO)
(74) Vertreter: Hofstetter, Alfons J.

(56) Entgegenhaltungen:
- US-A- 5 843 070
- US-A- 6 139 542
- US-A1- 2002 077 797
- US-A1- 2004 002 697
- US-B1- 6 299 309

## Beschreibung

Die vorliegende Erfindung betrifft ein Verfahren zur Steuerung eines augenchirurgischen Lasers für die Exzision bzw. Ablation eines definierten Hornhautvolumens eines menschlichen oder tierischen Auges.

Vorrichtungen und Verfahren zur Steuerung eines augenchirurgischen Lasers sind bekannt. So beschreibt die internationale Patentanmeldung WO 02/22003 eine Vorrichtung zur Bestimmung eines abzutragenden Bereichs von Hornhautgewebe, wobei das Volumen des abzutragenden Gewebes mit Hilfe einer pachymetrischen Vermessung des entsprechenden Bereichs der abzutragenden Hornhaut ermittelt wird. Die ermittelten pachymetrischen Daten dienen dabei zur Herstellung eines Betts innerhalb der Hornhaut zur Aufnahme einer entsprechenden Spenderhornhaut. Auch die US 6,551,306 beschreibt ein Verfahren und eine Vorrichtung zur Kontrolle des Tiefenabtrags auf der Hornhaut. Anhand von topographisch / pachymetrischen Daten wird während einer Operation ein entsprechender Laser gesteuert und kontrolliert. US 6 299 309 offenbart eine Vorrichtung und ein Verfahren zur Berechnung eines refraktiv wirkenden Ablationsvolumens auf Basis topographischer Daten.

Nachteilig an diesen bekannten Verfahren und Vorrichtungen ist jedoch, dass die bekannten Verfahren das zu exzisierende bzw. abladierende Volumen nur unzureichend berechnen und darstellen. Die mit den bekannten Verfahren ermittelten Werte zur Steuerung eines augenchirurgischen Lasers sind daher ebenfalls unzureichend und können daher nur eine Annäherung an das optimale zu exzisierende bzw. abladierende Volumen darstellen.

Es ist daher Aufgabe der vorliegenden Erfindung ein Verfahren zur Steuerung eines augenchirurgischen Lasers für die Exzision bzw. Ablation eines Hornhautvolumens eines menschlichen oder tierischen Auges bereitzustellen, welches einen für den jeweiligen Verwendungszweck optimiertes Exzisions- bzw. Ablationsvolumen der Hornhaut berechnet, darstellt und entsprechend optimierte Steuerungswerte für den Laser zur Verfügung stellt.

Gelöst wird diese Aufgabe durch ein Verfahren gemäß den Merkmalen des Anspruchs 1.

Vorteilhafte Ausgestaltungen sind in den Unteransprüchen beschrieben.

Ein erfindungsgemäßes Verfahren zur Steuerung eines augenchirurgischen Lasers für den Abtrag einer Hornhautschicht bei zum Beispiel einer Patienten- oder Spenderhornhaut eines menschlichen oder tierischen Auges umfasst die in Anspruch 1 offenbarten Schritte:
(a) Pachymetrische oder pachymetrisch-topographische Vermessung von zumindest einem Teilbereich der Hornhaut durch manuelle und/oder automatische Messdatenerfassung mittels eines Mess-Systems.
(b) Berechnung eines Volumenkörpers auf Grundlage der vermessenen Bereiche der Hornhaut durch volumenbeschreibende Funktionen oder Interpolation der in Verfahrensschritt a) ermittelten Messdaten und/ oder einer nachfolgenden Plausibilitätsprüfung der vorliegenden Daten,
(c) automatische und/oder manuelle Bereitstellung und Eingabe von Daten bezüglich der gewünschten Tiefe, des Durchmessers und der Geometrie der Hornhautexzision oder -ablation,
(d) Berechnung eines modifizierten Exzisions- oder Ablationsvolumens durch volumenbeschreibende Funktionen und/oder Interpolation des in Verfahrensschritt b) berechneten Volumenkörpers mit den Daten gemäß Verfahrensschritt c);
(e) Darstellung des modifizierten Exzisions- oder Ablationsvolumens durch volumenbeschreibende Funktionen und/oder Interpolation des in Verfahrensschritt d) berechneten Volumenkörpers;
(f) automatische und/oder manuelle Bereitstellung und Eingabe von Daten bezüglich der für die Exzision oder Ablation der Hornhaut verwendeten laser- und hornhautspezifischen Korrekturfaktoren;
(g) Verrechnung des in d) berechneten Exzisions- oder Ablationsvolumens mit den in f) erfassten Korrekturfaktoren und Darstellung des resultierenden Exzisions- oder Ablationsvolumens durch volumenbeschreibende Funktionen; und
(h) Export der in Verfahrensschritt g) berechneten Daten zur externen Weiterverarbeitung oder Berechnung einer Laserspot-Verteilung zur Erzeugung des in Verfahrensschritt g) berechneten Exzisions- oder Ablationsvolumens unter Berücksichtigung von gerätespezifischen Parametern von augenchirurgischen Lasern und Übergabe der berechneten Laser-Schusskoordinaten an den Laser.

Durch die Verwendung von volumenbeschreibenden Funktionen oder die Interpolation der in Verfahrensschritt a) ermittelten Messdaten erfolgt eine optimierte Berechnung des Volumenkörpers von zumindest einem Teilbereich der Hornhaut. Entsprechendes gilt für die Berechnung des modifizierten Exzisions- bzw. Ablationsvolumens in d) durch volumenbeschreibende Funktionen und/oder Interpolation des in Verfahrensschritt b) berechneten Volumenkörpers mit den Daten gemäß Verfahrensschritt c). Volumenbeschreibende Funktionen und/oder Interpolationen weisen deutliche Vorteile gegenüber der bisher verwendeten Subtraktionen des gewünschten Exzisions- bzw. Ablationsvolumens vom pachymetrisch ermittelten Volumenkörper, wie dies zum Beispiel in der WO 02/22003 geschieht, auf. Im Gegensatz zu allen bisher bekannten Verfahren verwendet das erfindungsgemäße Verfahren hornhaut- und laserspezifische Korrekturfaktoren zur Berechnung des resultierenden Exzisions- bzw.

Ablationsvolumens, welches vorteilhafterweise vor dem Export an den augenchirurgischen Laser noch mit den jeweiligen gerätespezifischen Parametern nachkorrigiert wird. Dadurch ist es möglich, nicht nur ein theoretisches, sondern auch ein in der Praxis optimales Exzisions- bzw. Ablationsvolumen zu erhalten. Bei den in Verfahrensschritt f) beschriebenen Korrekturfaktoren handelt es sich zum Beispiel um folgende laser- und hornhautspezifische Faktoren: Spotverhalten außerhalb des Fokus und Fluence, Ablationsverhalten innerhalb des Stromas, Verhalten des Stromas bei Photodisruption, maximale lokale Wiederholfrequenz, Anatomie- bzw. Stabilititätsbetrachtung aus topographischen und/oder pachymetrischen Daten, Einflüsse aus der Lagerung und dem Alter der verwendeten Spenderhornhaut. Bei den gerätspezifischen Parametern gemäß Verfahrensschritt h) kann es sich beispielhaft um folgende Parameter handeln: Scannerauflösung, Eye-Tracking-Geschwindigkeit, Beam-Shape, Fluence und Laserspotgröße.

In einer vorteilhaften Ausgestaltung des erfindungsgemäßen Verfahrens erfolgt vor dem Verfahrensschritt (h) eine Simulation des Laserabtrags zur Ermittlung der optimalen Schussverteilung bezogen auf das errechnete Exzisions- bzw. Ablationsvolumen. Zudem ist der Volumenkörper und/oder das modifizierte Exzisions- bzw. Ablationsvolumen und/oder das resultierende Exzisions- bzw. Ablationsvolumen zwei- oder dreidimensional darstellbar. Durch die Simulation ist eine Ermittlung der optimalen Hornhautexzision bzw. -ablation durch den Laser möglich. Des Weiteren ist es möglich, dass zu jedem Zeitpunkt der Exzisions- bzw. Ablationsvorgang noch vor der eigentlichen Behandlung simuliert und beobachtet werden kann.

In einer vorteilhaften Ausgestaltung des erfindungsgemäßen Verfahrens erfolgt die Berechnung und die Darstellung der Exzisions- bzw. Ablationsvolumens gemäß den Verfahrensschritten b), d), e) und g) durch Zernike-Koeffizienten, Matrizen, Punktkoordinaten, Best-fit - Methoden, kartesische oder Polar - Koordinaten, Vektoren bzw. Vektorkoordinaten oder Ähnliche. Durch die obengenannten mathematischen Darstellungsmöglichkeiten ergibt sich eine äußerst exakte Berechnung und Darstellung der genannten Hornhautvolumen.

In einer weiteren vorteilhaften Ausgestaltung des erfindungemäßen Verfahrens erfolg die pachymetrische Vermessung von zumindest einem Teilbereich der Hornhaut gemäß dem Verfahrensschritt a) mittels hochauflösenden Hornhautdickenmessgeräts, wie beispielsweise eines Scheimpflugsystems, Orbscan, OCT, Astramax Topographiegerätes oder eines Ultraschallbasierenden, vollflächigen Mess-Systems wie der Artemis. Es ist auch möglich, einen Ein-Punkt-Ultraschall-Pachymeter mit Ultraschallmesskopf zu verwenden. Dabei umfasst die pachymetrische Vermessung eine Vielzahl von Messpunkten auf der Hornhaut.

In einer weiteren vorteilhaften Ausgestaltung des erfindungemäßen Verfahrens erfolgt die Bestimmung des optimalen Exzisions- bzw. Ablationsvolumen durch eine Verrechnung von Pachymetrie- mit den Topographiedaten der Hornhaut (pachymetrisch / topographische Laserkorrektur). Aus dem Stand der Technik ist die alleinige Verwendung von Topographiedaten zur Bestimmung des Ablationsvolumens ohne gleichzeitige Betrachtung der jeweiligen Pachymetrie bekannt. Die Kombination aus Pachymetrie- und Topographiedaten ermöglicht eine wesentlich genauere Betrachtung der Auswirkung von lokal unterschiedlichen Pachymetriewerten auf die lokale Topographie und vermeidet somit eine zu starke lokale Stabilitätsschwächung der Hornhaut und einer damit einhergehenden Keratokonusausbildung (Hornhautauswölbung).

Die pachymetrische Vermessung der Hornhaut und die Berechnung des Volumenkörpers gemäß den Verfahrensschritten a) und b) dienen insbesondere zur Ermittlung von Fehlsichtigkeiten des Auges oder zur Ermittlung eines Transplantationsbetts für ein Hornhauttransplantat oder zur Bearbeitung eines Hornhauttransplantates.

Weitere Vorteile, Einzelheiten und Merkmale des erfindungsgemäßen Verfahrens werden anhand zweier in den Figuren dargestellter Ablaufdiagramme beispielhaft dargestellt. Es zeigen
- Figur 1: ein Ablaufdiagramm des erfindungsgemäßen Verfahrens zur Steuerung eines augenchirurgischen Lasers bei der Behandlung einer Patientenhornhaut; und
- Figur 2: ein Ablaufdiagramm des erfindungsgemäßen Verfahrens zur Steuerung eines augenchirurgischen Lasers bei der Behandlung einer Spenderhornhaut.

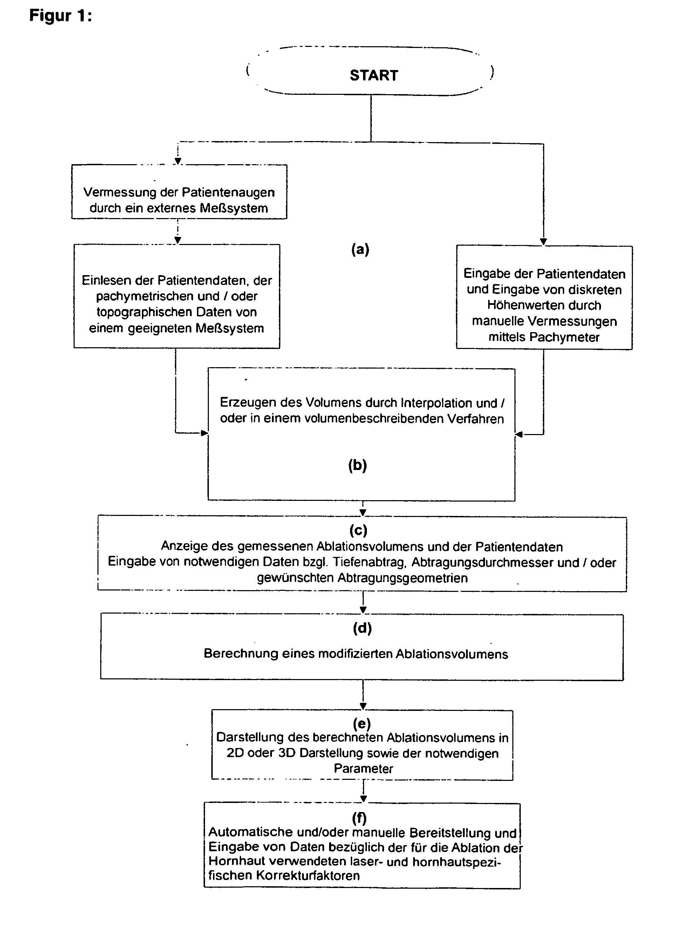

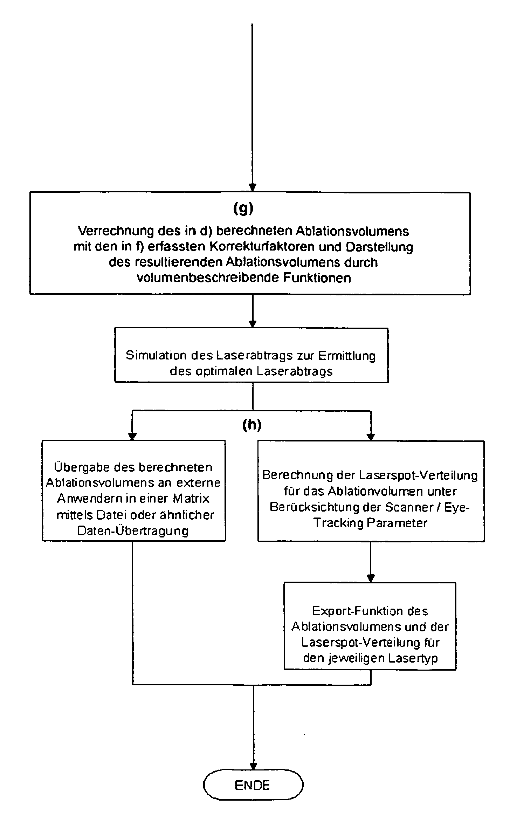

Figur 1 zeigt ein Ablaufdiagramm eines Verfahrens zur Steuerung eines augenchirurgischen Lasers bei der Behandlung einer Patientenhornhaut. Bei dem Laser kann es sich dabei um einen Excimer-Laser, einen Solid-State-Laser, einen fs-Laser, einen ps-Laser oder ganz allgemein um einen Schneidelaser bzw. Ablationslaser handeln. Man erkennt, dass in einem ersten Verfahrensschritt a) neben der pachymetrischen oder pachymetrisch-topographischen Vermessung von zumindest einem Teilbereich der Hornhaut die entsprechenden Daten entweder manuell oder durch Einlesen aus einem Diagnose-System zur Verfügung gestellt werden.

In dem folgenden Verfahrensschritt b) erfolgt die Berechnung und Darstellung des in a) gemessenen Volumenkörpers durch Interpolation oder volumenbeschreibende Funktionen.

Im Rahmen des Verfahrensschritts c) werden notwendige Daten wie gewünschte Tiefe, Durchmesser und Geometrie der Exzision bzw. Ablation eingegeben.

In dem folgenden Verfahrensschritt d) erfolgt die Berechnung des Exzisions- bzw. Ablationsvolumens unter Berücksichtigung der in c) eingegebenen Werte durch volumenbeschreibende Funktionen und/oder durch Interpolation.

Im Verfahrensschritt e) erfolgt die Darstellung des modifizierten Exzisions- bzw. Ablationsvolumens durch volumenbeschreibende Funktionen in 2D oder 3D und / oder Interpolation;

Im Verfahrensschritt f) erfolgt die automatische und/oder manuelle Bereitstellung und Eingabe von Daten bezüglich des für die Exzision bzw. Ablation der Hornhaut verwendeten Lasers, insbesondere die Bereitstellung und Eingabe von laser- und hornhautspezifischen Korrekturfaktoren;

In dem folgenden Verfahrensschritt g) erfolgt die Berechnung und Anzeige eines resultierenden Exzisions- bzw. Ablationsvolumens durch Verrechnung des theoretischen Exzisions- bzw. Ablationsvolumens aus Verfahrensschritt d) mit den laser- und hornhautspezifischen Korrekturfaktoren aus Verfahrensschritt f).

Vor dem abschließenden Verfahrensschritt (h) wird gemäß diesem Ausführungsbeispiel eine Simulation der Hornhautexzision bzw. -ablation zur Verifizierung des optimalen, laserspezifischen Exzisions- bzw. Ablationsverhaltens vor der tatsächlichen Hornhautexzision bzw. -ablation durchgeführt.

Schließlich erfolgt im abschließenden Verfahrensschritt (h) ein Export des berechneten Exzisions- bzw. Ablationsvolumens an externe Anwender zur externen Weiterverarbeitung oder eine direkte Berechnung der in Verfahrensschritt g) bereitgestellten Daten zur Berechnung der Laser-Schusskoordinaten unter Berücksichtigung der gerätespezifischen Parametern, wie zum Beispiel von Eye-tracking- bzw. Scanner-Parametern). Auch werden unterschiedliche Laser-Schussprofile wie zum Beispiel Schnittkontur- oder schichtweiser Volumenabtrag, Volumenbeschreibende und Schnittkonturbeschreibende Funktionen berücksichtigt. Daraus ergibt sich schließlich eine Bereitstellung einer Exportfunktion für das berechnete Exzisions- bzw. Ablationsvolumen und der Laser-Schusskoordinaten für den jeweiligen Lasertyp.

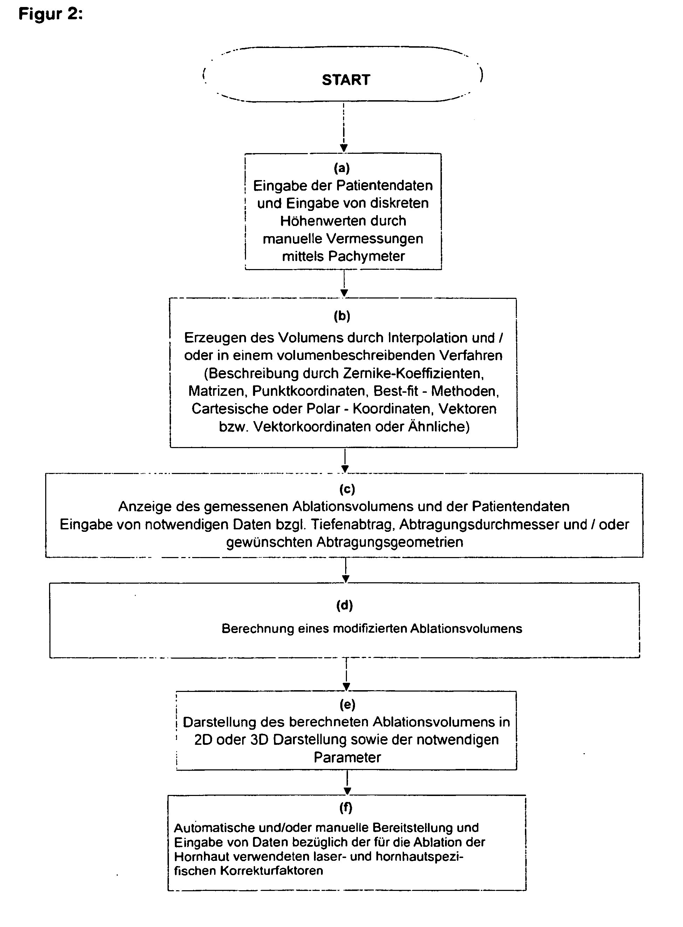

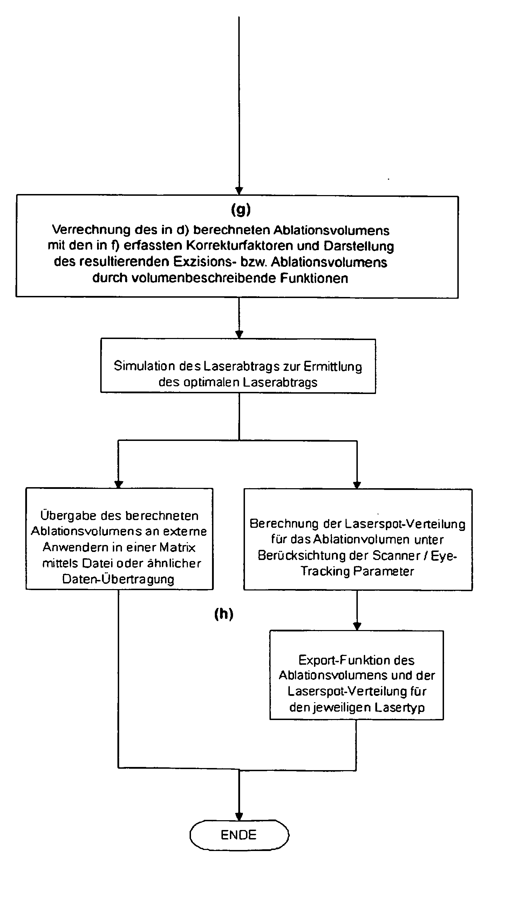

Figur 2 zeigt ein Ablaufdiagramm eines Verfahrens zur Steuerung eines augenchirurgischen Lasers bei der Behandlung einer Spenderhornhaut. Man erkennt, dass im Gegensatz zu dem in Figur 1 dargestellten Ausführungsbeispiel die spezifischen Patientendaten und insbesondere die Eingabe von diskreten Höhenwerten durch eine manuelle Vermessung mittels eines Fachymeters erfolgen. Die weiteren Verfahrensschritte entsprechen den in dem ersten Ausführungsbeispiel beschriebenen.

## Patentansprüche

1. Verfahren zur Steuerung eines augenchirurgischen Lasers für die Exzision oder Ablation eines Hornhautvolumens eines menschlichen oder tierischen Auges, wobei das Verfahren folgende Schritte umfasst:
(a) manuelle und/oder automatische Erfassung von Messdaten zur Hornhautdicke von zumindest einem Teilbereich der Hornhaut mittels eines Hornhautdickenmessgeräts,
(b) Berechnung eines Volumenkörpers zur Beschreibung der Dicke der vermessenen Bereiche der Hornhaut auf Grundlage der in Verfahrensschritt a) erfassten Messdaten zur Hornhautdicke durch volumenbeschreibende Funktionen oder durch Interpolation,
(c) automatische Bereitstellung und/oder manuelle Eingabe von Daten bezüglich der gewünschten Tiefe, des Durchmessers und der Geometrie der Hornhautexzision oder -ablation,
(d) Berechnung eines modifizierten Exzisions- oder Ablationsvolumens durch volumenbeschreibende Funktionen und/oder Interpolation des in Verfahrensschritt b) berechneten Volumenkörpers mit den Daten gemäß Verfahrensschritt c);
(e) Darstellung des modifizierten Exzisions- oder Ablationsvolumens durch volumenbeschreibende Funktionen und/oder Interpolation des in Verfahrensschritt d) berechneten Volumenkörpers;
(f) automatische Bereitstellung und/oder manuelle Eingabe von Daten bezüglich der für die Exzision oder Ablation der Hornhaut verwendeten laser- und homhautspezifischen Korrekturfaktoren;
(g) Verrechnung des in Verfahrensschritt d) berechneten Exzisions- oder Ablationsvolumens mit den in Verfahrensschritt f) erfasste Korrekturfaktoren und Darstellung des resultierenden Exzisions- oder Ablationsvolumens durch volumenbeschreibende Funktionen; und
(h) Export der in Verfahrensschritt g) berechneten Daten zur externen Weiterverarbeitung oder Berechnung einer Laserspot-Verteilung zur Erzeugung des in Verfahrensschritt g) berechneten Exzisions- oder Ablationsvolumens unter Berücksichtigung von gerätespezifischen Parametern von augenchirurgischen Lasern und Übergabe der berechneten Laser-Schusskoordinaten an den Laser.

2. Verfahren nach Anspruch 1,
**dadurch gekennzeichnet,**
**dass** vor dem Verfahrensschritt h) eine Simulation des Laserabtrags zur Ermittlung der optimalen Schussverteilung bezogen auf das errechnete Exzisions- oder Ablationsvolumen durchgeführt wird.

3. Verfahren nach Anspruch 1 oder 2,
**dadurch gekennzeichnet,**
**dass** die Berechnung oder Darstellung des ermittelten Exzisions- oder Ablationsvolumens gemäß den Verfahrensschritten b), d), e) und g) durch volumenbeschreibende Funktionen durch Zernike-Koeffizienten, Matrizen, Punktkoordinaten, Best-fit-Methoden, kartesische oder Polar-Koordinaten, Vektoren oder Vektorkoordinaten erfolgt.

4. Verfahren nach einem der Ansprüche 1 bis 3,
**dadurch gekennzeichnet,**
**dass** die Vermessung von zumindest einem Teilbereich der Hornhaut zusätzlich die Bestimmung und Vermessung der Topographie der Hornhautoberfläche umfasst.

5. Verfahren nach einem der vorhergehenden Ansprüche,
**dadurch gekennzeichnet,**
**dass** die berechneten Volumenkörper zwei- und/oder dreidimensional darstellbar sind.

## Claims

1. A method for controlling a laser for eye surgery for the excision or ablation of a corneal volume of a human or animal eye, respectively, wherein the method includes the following steps:
(a) manually and/or automatically acquiring measurement data concerning the thickness of at least one part of the cornea by means of a measurement device for measuring the cornea thickness;
(b) calculating a volume element for describing the thickness of the measured parts of the cornea on the basis of the measured data concerning the cornea thickness determined in method step a) using volume-describing functions or through interpolation;
(c) automatically providing and/or manually entering data concerning the desired depth, the diameter, and the geometry of the corneal excision or ablation, respectively;
(d) calculating a modified excision or ablation volume, respectively, using volume-describing functions and/or by interpolation of the volume element calculated in method step b) with the data according to method step c);
(e) representing the modified excision or ablation volume, respectively, using volume-describing functions and/or by interpolation of the volume element calculated in method step d);
(f) automatically providing and/or manually entering data concerning the correction factors specific to the laser and to the cornea used for the excision or ablation of the cornea, respectively;
(g) applying the correction factors acquired in method step f) to the excision or ablation volume calculated in method step d), respectively, and representing the resulting excision or ablation volume, respectively, using volume-describing functions; and
(h) exporting the data calculated in method step g) for external further processing or calculation of a laser spot distribution for generating the excision or ablation volume calculated in method step g), respectively, taking into consideration device-specific parameters of lasers for eye surgery and transferring the calculated laser shot coordinates to the laser.

2. The method according to claim 1,
**characterized in that**
a simulation of the laser ablation for determining the optimum shot distribution with respect to the calculated excision or ablation volume, respectively, is performed before method step (h).

3. The method according to claim 1 or 2,
**characterized in that**
the calculation or representation of the determined excision or ablation volume, respectively, according to the method steps b), d), e) and g) by volume-describing functions is effected by Zernike coefficients, matrices, point coordinates, best fit methods, Cartesian or polar coordinates, vectors or vector coordinates, respectively.

4. The method according to any one of claims 1 to 3,
**characterized in that**
the measurement of at least one part of the cornea is effected by the determination and measurement of the topography of the corneal surface.

5. The method according to anyone of the preceding claims,
**characterized in that**
the calculated volume elements can be represented two-dimensionally and/or three-dimensionally.

## Revendications

1. Procédé destiné à la commande d'un laser ophtalmologique pour l'excision ou l'ablation d'un volume de la cornée de l'oeil humain ou animalier, ledit procédé comprenant les étapes suivantes :
(a) la saisie manuelle et/ou automatique des données de mesure concernant l'épaisseur de la cornée sur au moins une partie de celle-ci au moyen d'un pachymètre ;
(b) le calcul d'un corps volumique afin de décrire l'épaisseur des parties mesurées de la cornée sur la base des données de mesure relative à l'épaisseur de la cornée relevées à l'étape a) du procédé, et ce par des fonctions décrivant le volume ou par interpolation ;
(c) la mise à disposition automatique et/ou la saisie manuelle de données concernant la profondeur désirée, le diamètre et la géométrie de l'excision ou de l'ablation cornéenne;
(d) le calcul d'un volume d'excision ou d'ablation modifié au moyen de fonctions décrivant le volume et/ou de l'interpolation du corps volumique calculé à l'étape b) du procédé en faisant appel aux données selon l'étape c) ;
(e) la représentation du volume d'excision ou d'ablation par des fonctions décrivant le volume et/ou l'interpolation du corps volumique calculé à l'étape d) du procédé;
(f) la mise à disposition automatique et/ou la saisie manuelle des données concernant les facteurs de correction spécifiques au laser et à la cornée utilisés pour l'excision ou l'ablation de la cornée ;
(g) la compensation du volume d'excision ou d'ablation calculé à l'étape d) du procédé et des facteurs de correction recensés à l'étape f), ainsi que la représentation du volume d'excision ou d'ablation à travers de fonctions décrivant le volume, ainsi que :
(h) l'exportation des données calculées à l'étape g) du procédé afin d'en permettre un traitement externe ou le calcul d'une répartition des spots du laser pour produire le volume d'excision ou d'ablation calculé à l'étape g) du procédé, en prenant en compte les paramètres spécifiques des appareils laser destinés à la chirurgie oculaire, et transmission des coordonnées d'impact calculées au laser.

2. Procédé selon la revendication 1,
**caractérisé en ce qu'**
il est réalisé une simulation de l'ablation au laser avant l'étape h) du procédé afin de déterminer la répartition optimale des tirs en fonction du volume d'excision ou d'ablation calculé.

3. Procédé selon les revendications 1 ou 2,
**caractérisé en ce que**
le calcul ou la représentation du volume d'excision ou d'ablation établi en vertu des étapes b), d), e) et g) du procédé se fait au moyen de fonctions décrivant des volumes à l'aide de coefficients de Zernike, de matrices, de coordonnées ponctuelles, de méthodes best-fit, de coordonnées cartésiennes ou polaires, de vecteurs ou de coordonnées vectorielles.

4. Procédé selon l'une des revendications 1 à 3,
**caractérisé en ce que**
la mesure d'au moins une partie de la cornée comprend en outre l'établissement et le relevée de la topographie de la surface cornéenne.

5. Procédé selon l'une des revendications ci-devant,
**caractérisé en ce que**
les corps volumiques calculés se prêtent à la présentation en 2 et/ou 3D.
